(19) **Europäisches Patentamt / European Patent Office / Office européen des brevets**

(11) **EP 4 138 641 B1**

(12) **EUROPEAN PATENT SPECIFICATION**

(45) Date of publication and mention
of the grant of the patent:
**17.04.2024 Bulletin 2024/16**

(21) Application number: **21724200.7**

(22) Date of filing: **23.04.2021**

(51) International Patent Classification (IPC):
*A61B 5/00* *(2006.01)*    *A61B 5/107* *(2006.01)*
*A61B 5/20* *(2006.01)*

(52) Cooperative Patent Classification (CPC):
**A61B 5/4878; A61B 5/0064; A61B 5/0077;
A61B 5/1072; A61B 5/1075; A61B 5/1079;
A61B 5/208**

(86) International application number:
**PCT/EP2021/060760**

(87) International publication number:
**WO 2021/214335 (28.10.2021 Gazette 2021/43)**

(54) **MONITORING FLUID BALANCE**

ÜBERWACHUNG DES FLÜSSIGKEITSHAUSHALTS

SURVEILLANCE D'ÉQUILIBRE DE FLUIDES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **24.04.2020 EP 20171259**

(43) Date of publication of application:
**01.03.2023 Bulletin 2023/09**

(73) Proprietor: **Elmedix NV**
**3001 Leuven (BE)**

(72) Inventors:
• **VAN DEN BOSSCHE, Johan**
**3210 Linden (BE)**

• **BOGERS, Johannes**
**2530 Boechout (BE)**
• **BRANCATO, Luigi**
**3010 Leuven (BE)**

(74) Representative: **IPLodge bv**
**Technologielaan 9**
**3001 Heverlee (BE)**

(56) References cited:
GB-A- 2 564 243     US-A1- 2006 235 353
US-A1- 2016 235 354     US-A1- 2018 085 510
US-A1- 2018 235 539

## Description

## Technical field of the invention

The present invention relates to the field of healthcare monitoring, and particularly to the continuous or periodic monitoring of physiological parameters over time. The present invention specifically relates to a device for monitoring the fluid balance of an animal or human body.

## Background of the invention

Under normal conditions, a tight control of the amount of water in the body is maintained as one of the aspects of homeostasis. This fluid balance is necessary to keep concentrations of electrolytes within tolerance limits. In general terms, the body aims to balance the amount of water taken in with the amount of water that is lost through urine and feces, as well as via respiration and perspiration. An imbalance of the volume of water stored in the body, and typically also of the electrolyte concentrations associated therewith, can produce mild symptoms, such as fatigue or headaches, as well as serious complications. For example, a sudden increase in the water volume can cause hyponatremia, which can be fatal.

The human or animal body has a remarkable capacity for maintaining a healthy fluid balance, i.e. in which the body fluids, such as blood, interstitial fluid and intracellular fluid, are kept at in normal volume range. Maintaining this balance requires a frequent replenishment of fluids, primarily via the intake of water or other suitable liquids. It is well-known that, for example, humans can only survive a few days without water, while survival without food over substantial periods of time is possible. For example, the world record for survival without food, except for the intake of non-caloric beverages, vitamins and electrolytes, stands at 382 days.

While dehydration due to an insufficient intake of water or other beverages is obviously the most dominant risk factor for developing a fluid imbalance, diarrhea is another important factor that threatens healthy fluid volume and electrolyte levels.

Sweating and/or loss of fluid via respiration are also important risk factors. The latter factors are typically associated with exposure to a high temperature and/or and environment, and the effect of such exposure can rapidly exacerbate over time.

An accurate measure of fluid balance can be important in various medical scenarios. For example, illness can involve an elevated body temperature, vomiting, diarrhea and/or hemorrhaging, all of which can reduce the amount of fluid available to the body, i.e. causing a negative fluid balance. The kidneys play an essential role in managing the fluid balance, and, therefore, conditions that reduce the function of the kidneys, including kidney diseases, hypotension and/or other cardiovascular disfunctions, may cause an abnormal retention of fluid, i.e. a positive fluid balance. A direct disfunction of the renin-angiotensin-aldosterone system is obviously another example of a medical condition in which the fluid balance needs to be monitored and, possibly, actively corrected.

Monitoring of the fluid balance can thus be required, or at least desirable, under various conditions. This may be particularly the case in hyperthermia treatments. In such treatment, hyperthermia is deliberately induced by drugs and/or medical devices, for example as a primary or adjuvant treatment of cancer. For example, the entire body may be heated to (e.g.) 41.5 °C to overheat cancerous cells. This approach may be particularly useful for treating cancers that are widely distributed in the body. It shall be clear that the elevated temperature imposed by such treatment implies a risk of causing a negative fluid balance. Moreover, this treatment may require sedation or anesthesia, or the use thereof may be at least highly preferred. Under general anesthesia, the natural responses of the body to counteract the artificially imposed elevated temperature can be advantageously reduced or avoided. Furthermore, such treatment would be considerably unpleasant, possibly even unbearable, when the patient remains conscious, particularly for a treatment that lasts many hours, possibly even more than a day. However, under anesthesia, the homeostatic control mechanisms of the body for maintaining its fluid balance may also be compromised, further adding to the need of a system for monitoring and managing the fluid balance.

It is known in the art that the fluid balance of burn or major trauma patients may need to be monitored closely, since fluid losses may be serious in such cases. Maintenance of an adequate urine output of approximately 5-100 cc per hour and per 10 kg body weight may be considered as an important parameter in the treatment. For example, it is known in the art to periodically weigh the urine to monitor the fluid balance.

For example, US 4,291,692 discloses a closed loop system for controlled infusion of fluid into a patient based on real time measurement of urine output. A real time urine flow rate is compared with a preset, desired flow rate to develop an error signal which is processed by a computer. A proportional-integral-derivative (PID) control algorithm is used to govern the infusion of fluids into the patient.

However, it is a disadvantage of such prior-art approaches that an optimal urine output needs to be presupposed, and that a fluid intake is not optimized to achieve a net neutral fluid balance, but to optimize this presupposed optimal urine output. It is a further disadvantage that fluid losses via other routes than via urine are ignored, while such other fluid losses may be substantial, e.g. respiratory and perspiratory losses in a hyperthermia treatment.

US 4,449,538 discloses another example of an electronic body fluid accounting system. A microcomputer receives data derived from a plurality of fluid input and output channels which together represent the prevailing fluid input and output sources associated with the patient. The input channels include intravenous liquid flows, e.g. of different liquids. The output channels include the amounts of fluids discharged through a urinary or other catheter, an amount of exudate drawn from a surgical site, and an amount of blood taken from the patient by sponges. On the basis of the data, the computer yields a fluid intake total and a fluid outtake total, which totals are displayed to indicate the prevailing fluid balance. This system may be particularly suitable to assist an anesthesiologist during surgical interventions.

However, unlike the aforementioned prior-art systems, this illustrative prior-art system merely provides a convenient registration system to keep track of the patient's fluid balance, without actively controlling a fluid flow provided to the patient in order to attain an optimum, e.g. a urine production in a target range. It is a further disadvantage that this system does not provide a solution to keep track of fluid losses via respiration and/or perspiration. The latter may be negligible in a typical surgical setting, but may become substantial under specific conditions, such as unfavorable ambient conditions, e.g. in a field hospital or triage post, or a treatment in which an elevated ambient temperature is essential, e.g. a hyperthermia treatment.

US 5,522,805 discloses an approach that is substantially similar to the fluid accounting system of US 4,449,538.

Excessive fluid retention, generally referred to as edema, is an indication of a disrupted homeostatic body response. When the body fails to prevent the build-up of fluids, this is an important indication that a healthy concentration of electrolytes and healthy body pressures (e.g. blood pressure) cannot be maintained, or excess fluid cannot be removed adequately. It is important that such fluid retention is detected as soon as possible. For example, when swelling and/or breathing difficulties become apparent to a physician, the condition of the patient may already be deteriorating rapidly, e.g. possibly leading to cardiac heart failure.

Devices are known in the art to detect edema using impedance measurements. US 5,876,353 discloses an implantable impedance measurement device that can provide a graded indication of edema, i.e. indicating the severity of the condition, and which can be used for monitoring over a protracted period of time. The transthoracic impedance measurement is used as an indicator of the level of edema, and the detection and/or quantification of edema can be further enhanced by monitoring the respiratory rate. However, it is a disadvantage of this approach, and related prior-art methods, that only edema is detected, e.g. without considering the context of a net fluid balance. The document US 2018/085510 A1 represents further relevant prior art with regard to the present disclosure.

## Summary of the invention

It is an object of the present invention to provide good means to monitor and/or correct the fluid balance of the human or animal body, e.g. during a medical procedure, such as a hyperthermia treatment. It is a further object to monitor the body for symptoms of fluid retention, i.e. of edema.

It is an advantage of embodiments of the present invention that, by continuously (or at least periodically, e.g. frequently) weighing the subject, fluid losses or gains (and/or a temporal derivative thereof) in the body can be accurately estimated.

It is an advantage of embodiments of the present invention that feedback, e.g. an alarm, can be generated when a fluid loss or gain exceeds a predetermined threshold and/or when local fluid retention is detected.

It is an advantage of embodiments of the present invention that a flow rate of a fluid delivery device, e.g. an intravenous fluid delivery device such as an infusion pump for providing an intravenous solution to the patient, can be automatically adjusted to reduce a detected net fluid gain or loss, e.g. optimized so as to strive for a net zero balance.

It is an advantage of embodiments of the present invention that a weight of a patient can be closely monitored, in which this weight can be used for estimating a fluid balance of the body. It is a further advantage that the measured weight of the patient can be used for other purposes as well, such as a parameter in a simulation, prediction and/or estimation of heat flow in and out of the body.

It is an advantage of embodiments of the present invention that an automated system (resp. method) is provided to determine fluid losses of the body, including fluid losses via urine, but not necessarily limited thereto.

It is an advantage of embodiments of the present invention that a fluid may be provided to the patient, e.g. via infusion, to compensate for fluid losses, e.g. to automatically maintain a net zero balance of fluid. For example, a drop volume of an intravenous fluid delivery device may be automatically adjusted as function of determined fluid losses of the body.

It is an advantage of embodiments of the present invention that a net zero fluid balance can be maintained, e.g. during a hyperthermia treatment.

It is an advantage of embodiments of the present invention that a net neutral fluid balance can be achieved without relying on a presupposed optimum of urine output.

It is an advantage of embodiments of the present invention that fluid losses via other routes than via urine can be taken into account to achieve a net neutral fluid balance. It is a further advantage that fluid losses via respiration and perspiration can be actively and accurately compensated, which may be particularly important when a patient is exposed to a hot and/or arid environment, and even more so when the patient is sedated or anesthetized. The latter may be particularly relevant for medical treatments or recovery, e.g. of burn or trauma victims, in a hot and/or arid environment, e.g. in a field hospital in an unfavorable climate, or for specific treatments, such as hyperthermia treatments.

It is an advantage of embodiments of the present invention that edema can be detected, which indicates a potentially critical failure of the autoregulation of fluid balance, in addition to the accurate determination of a fluid balance of the body. It shall be clear that the determination of a fluid balance, e.g. a net fluid gain or loss, a feedback control of the fluid balance, e.g. to aim for a zero net balance, and the detection of fluid retention, as indicator of a serious failure of the body to maintain its fluid balance, are substantially different, yet highly complementary, features of embodiments of the present invention.

The above objective is achieved by a device and method in accordance with embodiments of the present invention.

Other objects and advantages of the invention will become apparent from the following description, drawings, and/or the claims appended hereto.

In a first aspect, the present invention relates to a monitoring device for monitoring the fluid balance according to claim 1.

The device in accordance with embodiments of the present invention may comprise a fluid retention detector for detecting edema in at least a part of the body. The processor is furthermore adapted to detect a condition of edema based on a signal provided by the fluid retention detector.

In a device in accordance with embodiments of the present invention, the body weight sensor may comprise at least one load cell, integrated in or operably connected to a support structure for supporting the body in use of the device.

A device in accordance with embodiments of the present invention may comprise an alarm to alert an operator, in which the processor is adapted to activate the alarm when a fluid loss or gain exceeds a predetermined threshold and/or when the condition of edema is detected.

In a device in accordance with embodiments of the present invention, the body weight sensor comprises at least three load cells, preferably four load cells, integrated in or operably connected to a support structure for supporting the body, wherein the support structure essentially forms a plane, being a flat surface in two dimensions X and Y. The load cells are typically each integrated in or operably attached to a leg of the support structure contacting the ground.

In a device in accordance with embodiments of the present invention, the processor 5 is adapted for determining the center of gravity of the body held by the support structure by use of data provided by said at least three load cells, preferably four load cells.

In a device in accordance with embodiments of the present invention, the processor may be adapted to control the fluid delivery device so as to block the delivery of fluid to the body when a fluid gain exceeds a predetermined threshold and/or the condition of edema is detected.

In a device in accordance with embodiments of the present invention, the fluid retention detector may comprise a range detector for determining a distance from at least a point of reference to a plurality of points on the surface of the body. The range detector may form a three-dimensional imaging system. The processor may be adapted for determining a volume of the body based on data provided by the range detector.

in a device in accordance with embodiments of the present invention, the range detector may comprise at least two cameras. The processor may be adapted for determining an image depth in images obtained by the cameras, for generating a three-dimensional contour model of the body based on the determined image depth and for determining a volume of the body based on the three-dimensional contour model as indicative of edema..

in a device in accordance with embodiments of the present invention, the range detector may comprise at least one laser light source for illuminating the body, and a sensor for measuring the reflected light. For example, the range detector may also comprise a scanning system for scanning the laser light beam(s) emitted by the at least one laser light source over at least a part of the surface of the body while measuring the reflected light for a plurality of scanned points. The processor may be adapted for collecting point cloud data of the body based on measured reflected light for the plurality of scanned points, for generating a three-dimensional contour model of the body based on the point cloud data and for determining a volume of the body based on the three-dimensional contour model as indicative of edema.

In a device in accordance with embodiments of the present invention, the fluid retention detector may comprise an element for enclosing a part of the body and a sensor module, which is integrated in or operably attached to the element and adapted for generating a signal indicative of a circumference of the body part.

In a device in accordance with embodiments of the present invention, the sensor module may comprise a capacitive, inductive or resistive strain sensor, and/or a linear variable differential transformer.

In a device in accordance with embodiments of the present invention, the fluid retention detector may comprise a further sensor to provide a further physiological parameter measurement.

In a device in accordance with embodiments of the present invention, the fluid retention detector may comprise multiple parts for being positioned around different body parts and for generating separate signals indicative of swelling of each of the different body parts.

In a device in accordance with embodiments of the present invention, the fluid delivery device may comprise an intravenous pump.

In a device in accordance with embodiments of the present invention, the fluid delivery device may be adapted to generate a feedback signal indicative of a volume, a flow velocity or a flow rate of the fluid that is supplied.

In a device in accordance with embodiments of the present invention, the urine output sensor may comprise an optical sensing chamber, an ultrasonic transceiver, measuring head, and/or a weighing system.

In a second aspect, the present invention relates to a hyperthermia treatment system according to claim 14.

Further disclosed but not claimed is a method for monitoring the fluid balance of an animal or human body. The method comprises weighing the body using a body weight sensor, determining a urine output of the body using a urine output sensor, determining a total fluid outtake of the body, and optionally also a specific fluid outtake due to perspiration and respiration, by taking the determined body weight and the determined urine output into account. The method comprises regulating a volume, flow velocity or flow rate of a fluid delivered to the body using a fluid delivery device so as to maintain a predetermined fluid balance in the body. Optionally, the method further comprises detecting a condition of edema in at least a part of the body based on a signal provided by a fluid retention detector.

These and other aspects of the present invention will be apparent from and elucidated with reference to the embodiment(s) described hereinafter.

The independent and dependent claims describe specific and preferred features of the invention. Features of the dependent claims can be combined with features of the independent claims and with features of other dependent claims as deemec appropriate.

Brief Description of the Drawings

FIG 1 illustrates a device in accordance with embodiments of the present invention.
FIG 2 illustrates a fluid retention detector for use in a device in accordance with embodiments of the present invention.

The drawings are schematic and non-limiting. Elements in the drawings are not necessarily represented on scale, e.g. an element may be exaggerated for illustrative purposes or reduced in scale to keep the drawing clear and comprehensible. The present invention is not necessarily limited to the specific embodiments of the present invention as shown in the drawings. Reference signs in the claims shall not be construed as limiting the scope. In different drawings, the same reference signs may refer to the same or analogous elements.

Detailed description of embodiments

The present invention is only limited by the attached claims, notwithstanding the exemplary embodiments described hereinbelow. The attached claims are hereby explicitly referred to in this detailed description, in which each claim, and each combination of claims as allowed for by the dependency structure defined by the claims, forms a separate embodiment of the present invention.

The word "comprise," as used in the claims, is not limited to the features, elements or steps as described thereafter, and does not exclude additional features, elements or steps. This therefore specifies the presence of the mentioned features without excluding a further presence or addition of one or more features.

Ordinal references, such as first, second and the like, in the description and/or in the claims may be used to discern similar elements and do not necessarily define a sequence, either temporally, spatially, in ranking or in any other manner. Such terms may be interchangeable under appropriate circumstances and embodiments of the invention may relate to other sequences than explicitly described or illustrated herein.

Spatial references, such as top, bottom, on, under and the like, in the description and/or in the claims are used for descriptive purposes and not necessarily only for describing relative positions. It shall be clear that embodiments may relate to other positional arrangements of elements described using such spatial references, unless the relative positioning would be necessary for achieving the desired technical effect, i.e. for solving the underlying objective technical problem, as would be evident to the skilled person. Therefore, it is clear that such terms are interchangeable under appropriate circumstances and that embodiments of the present invention may be capable of operation in other orientations than described or illustrated herein.

In this detailed description, various specific details are presented. Embodiments of the present invention can be carried out without these specific details. Furthermore, well-known features, elements and/or steps are not necessarily described in detail for the sake of clarity and conciseness of the present disclosure.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments. References to "embodiments" or "in embodiments" are to be interpreted in the same way.

Various features of the invention may be grouped together in a single embodiment, figure, or description thereof for the purpose of streamlining the disclosure and aiding in the understanding of inventive aspects. This is not to be interpreted as reflecting an intention that the claimed invention requires more features than are expressly recited in each claim. Rather, as the following claims reflect, inventive aspects may lie in less than all features of a single foregoing disclosed embodiment as explicitly described in the description. Thus, the claims following the detailed description are hereby expressly referred to in this detailed description, with each claim standing on its own as a separate embodiment of this invention.

Furthermore, while some embodiments described herein include some but not other features included in other embodiments, and form different embodiments, as would be understood by those in the art.

In the description provided herein, numerous specific details are set forth. However, it is understood that embodiments of the invention may be practiced without these specific details. In other instances, well-known methods, structures and techniques have not been shown in detail in order not to obscure an understanding of this description.

In a first aspect, the present invention relates to a patient monitoring device for monitoring the fluid balance of an animal or human body, e.g. during a therapeutic or surgical procedure.

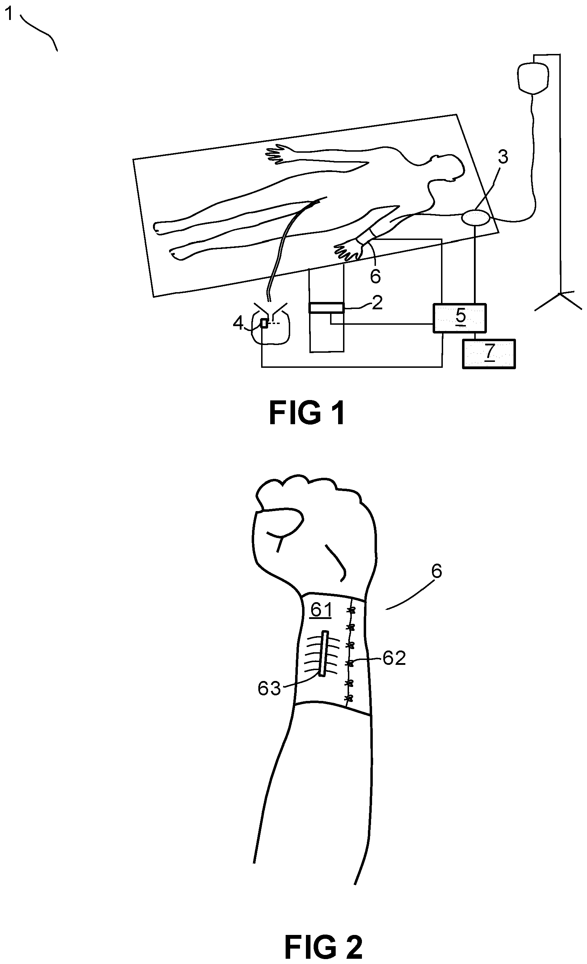

FIG 1 shows an illustrative device 1 in accordance with embodiments of the present invention. The device 1 comprises a body weight sensor 2 for periodically weighing the body, e.g. continuously or at least frequently weighing the body. For example, the body weight sensor may be adapted for sampling the body weight at a predetermined sampling frequency, e.g. at least once per minute, e.g. at least 6 times per minute, e.g. a sampling frequency in the range of 0.1 Hz to 1000 Hz, e.g. in the range of 1 Hz to 50 Hz. The body weight sensor may comprise a load cell (e.g. a force sensor). For example, the body weight sensor, e.g. the load cell, may be integrated in or operably attached to a support structure for supporting the body in use of the device. For example, such support structure may comprise an operating table, patient couch, or similar structure for holding the body of the patient. For example, an operating table may comprise one or more of such sensors, e.g. load cells. For example, the body may be, in operation, supported by the support structure, which may comprise one or more legs (e.g. four legs) contacting the ground for support. Each of said one or more legs may comprise a load cell, such that the weight of the body can be determined by analyzing measurement(s) obtained from the load cell(s).

In another illustrative embodiment, the body weight sensor 2 may comprise a floor scale or platform scale, e.g. a weight sensor system integrated in a (typically flat) surface element forming a floor. It is an advantage that the weight of the body can be conveniently monitored by (e.g. periodically, continuously or frequently) determining the total weight supported by the floor or platform scale. For example, the body can be positioned on a couch, a bed, an operating table or similar patient support device, which can be placed on the floor or platform scale to monitor the weight of the body in accordance with embodiments of the present invention. As is known in the art, such couch, bed or other patient support device may be mobile, e.g. may comprise wheels or other transport means such that the support device, preferably together with the supported body, can be easily transported, i.e. placed on the floor scale or platform scale to monitor the weight of the body.

The device 1 comprises a fluid delivery device 3 for providing a controllable supply of fluid to the body. The fluid may comprise distilled water, blood plasma, an isotonic saline solution, or a similar suitable fluid for replenishing the body fluids. The fluid may also comprise minerals, vitamins, other micronutrients, macronutrients and/or medication.

For example, the fluid delivery device 3 may comprise an intravenous pump. A volume or volume rate, e.g. a flow magnitude, of the flow of fluid delivered to the body may be, in operation, controlled by the processor 5. The fluid delivery device may be adapted to receive a control signal, and to regulate a volume, flow velocity or flow rate of the fluid based on the control signal. The fluid delivery device may also be adapted to generate a feedback signal indicative of a volume, a flow velocity or a flow rate of the fluid that is supplied. However, it shall be understood that such feedback signal may not be necessary when the fluid delivery device delivers a flow of fluid to the subject that can be accurately determined as function of the control signal.

The fluid delivery device 3 may thus comprise a pump, e.g. a peristaltic pump. The fluid delivery device may comprise an intravenous delivery device and/or an oral fluid dispenser. For example, such delivery devices may be described in US 2001/051788.

The device 1 comprises a urine output sensor 4 for determining the urine output of the body. For example, the urine output sensor may be adapted to measure the volume, flow velocity or flow rate of urine produced by the body. For example, the urine output sensor 4 may comprise a urine drop counter for counting drops of a substantially uniform volume.

For example, the urine output sensor 4 may comprise an optical sensing chamber to detect the drops. A flow restriction may ensure that droplets of substantially uniform size are created. In some embodiments, the flow restriction may be adjustable, e.g. manually or automatically controlled. For example, the diameter of a pinch tube may be adjustable to regulate the drop size. A similar approach can be used to control the volume of a liquid supplied to the body, e.g. in the fluid delivery device 3. An example of such approach for a urinary output monitor and/or a gravity fed volumetric controller in an intravenous (IV) delivery system can be found in US4504263.

The urine output sensor 4 may comprise a urine container for collecting urine from a urinary catheter. The sensor 4 may comprise an ultrasonic transceiver, mounted in or at the top of the container, for measuring the height of the urine collected in the container, e.g. as described in US4448207. In another example, a transducer may be mounted in the floor of the container, e.g. as described in US4658834.

In another example, the urine output sensor 4 may be adapted for measuring the urine flow from the subject. For example, a measuring head may be mounted in a funnel to measure the urine flow, e.g. as described in US5176148.

The urine output sensor 4 may comprise a weighing system for indirectly determining the amount of urine collected from the body by weighing a receptacle in which the urine is collected. Likewise, the amount of fluid supplied to the body by the fluid delivery system can be monitored, e.g. for verification purposes of the amount supplied in response to a control signal, by weighing the container in which the fluid to supply to the body is stored. Thus, the infusion of intravenous fluid and/or the amount of urine produced by the body can be monitored by weighing respective containers. It shall be clear that temporal differencing (e.g. a discrete temporal derivative) of such weighed receptacles can be used to obtain a measurement of momentary fluid intake and/or outtake ('outtake' referring to the sum of bodily fluids lost via various paths, such as respiration, perspiration, urine production, etc.).

The device may comprise a fluid retention detector 6 for detecting edema, i.e. for detecting an abnormal fluid retention, in at least a part of the body.

Referring to FIG 2, the fluid retention detector 6 may comprise a strip or, generally, an (e.g. elongate) element 61 for enclosing a part of the body, such as around a limb, e.g. around an arm, a wrist, a leg, a knee or an ankle, around the upper torso and/or around the lower torso.

The strip thus may comprise a stretchable component (i.e. in which at least a part thereof is stretchable) adapted for fitting securely around the body part. For example, the strip may form a closed loop, or the fluid retention detector may comprise a fastener(s) 62 to form a closed loop of the strip around the body part. The element for enclosing the body part may comprise a band, a belt, a sleeve, a garment and/or a brace.

The fluid retention detector may comprise a sensor module 63, e.g. forming part of the strip or attached to the strip or element. The sensor module may be adapted to generate a signal indicative of a circumference of the body part.

The sensor module may comprise a strain or stretch gauge, such as a capacitive, inductive or resistive strain sensor. Thus, the sensor module may generate a signal representative of a force caused by stretching the strip or element, and thus may be correlated with the circumference of the body part when worn. For example, the fluid retention detector may comprise a linear variable differential transformer. Such strain or stretch gauge may be adapted for detecting an enlargement of a contour (circumference) of the body part, indicative of a subdermal retention of fluid.

The fluid retention detector 6 may comprise a stretchable sensor patch. This patch may comprise an elastic film layer and at least one elastic measurement strip attached to the elastic film layer. Such measurement strip may comprise a capacitive strip with a dielectric electro-active polymer and/or an elastic resistive filament with a thermoplastic elastomer, such that a capacitance and/or a resistance changes as a function of a stretch of the measurement strip. An example of such stretchable sensor patch is disclosed in EP 3185757.

As another example, the fluid retention detector 6 may comprise a pressure sensor. For example, the fluid retention detector may comprise an actuator for compressing at least a point on the skin of the body, and, e.g. after a predetermined time interval, releasing the applied pressure. The pressure sensor may determine a measure indicative of relaxation of the tissue after the release of the applied pressure, as indicative of (a severity of) edema.

In another example for illustrating the use of a pressure sensor to determine edema, a cup or similar chamber may, in use, contact the skin. This chamber may be at a negative relative pressure, such that the skin is sucked into the chamber. The pressure sensor, in or attached to the chamber, may thus measure a pressure caused by the elasticity of the skin (and in equilibrium with the pressure in chamber). Thus, the elasticity of the skin can be determined, which may be used as a quantity indicative of edema.

The fluid retention detector 6 may also comprise an electrical sensor for determining an electrical property of the skin, such as an impedance, e.g. a resistance, of the skin. Such impedance may be used to determine a hydration level of the skin, as known in the art.

The fluid retention detector 6 may also comprise an optical sensor (e.g. and a suitable light source, e.g. a laser diode) for determining an optical property of the skin. For example, a light transmission factor, e.g. for a fixed wavelength of light and/or as variable function of wavelength, may be used to determine a hydration level of the skin. For example, near infrared light absorption may be used as a quantity that is indicative of edema. Likewise, a quantity related to light reflection, or other optical property, may be used.

Advantageously, the fluid retention detector 6, e.g. particularly when comprising a strip or other elongate element for fitting securely around a body part, may comprise a further sensor(s) to provide a physiological parameter measurement. For example, a pulse, a heart range, a blood pressure and/or a blood oxygenation sensor may be cointegrated with the fluid retention detector.

The fluid retention detector may comprise multiple parts for being positioned around different body parts and for generating separate signals indicative of swelling of each of the different body parts. Thus, advantageously, a distribution of fluid retention can be determined. Such multiple parts may be separate elements, or may be integrated in a single element, e.g. a garment.

The fluid retention detector 6 may also comprise a range detector for determining a distance from at least a point of reference to a plurality of points on the surface of the body, e.g. at a plurality of points on the body. The range detector may form an imaging system, e.g. a three-dimensional imaging system. Thus, a volume of the body may be estimated, which can be used to detect swelling due to edema.

For example, the fluid retention detector may comprise at least two cameras configured such as to determine an image depth in images obtained by the cameras. For example, the at least two cameras may form a stereographic imaging system. Preferably, the at least two cameras may comprise at least pairs of cameras at a plurality of locations around the body and aimed at the body, such as to enable the processor to construct a (e.g. substantially real-time) surface model of the body.

In another example, the range detector may comprise at least one laser light source for illuminating the body and a sensor for measuring the reflected light. The range detector may be adapted for detecting a time of flight (laser light return time) and/or a triangulation measurement of the light reflected by the body. The range detector may comprise a scanning system, such as to scan the laser light beam(s) emitted by the at least one laser light source over at least a part of the surface of the body while detecting the relevant return light parameter(s) in a plurality of scanned points. For example, the range detector may comprise a light detection and ranging (LIDAR) system or another suitable laser scanning system.

Thus, a laser radar system may be controlled to collect point cloud data of the body, e.g. at different angles, and a 3D contour model may be generated on the basis of this point cloud data, e.g. by the processor 6. Furthermore, the volume of the body may be determined from the 3D surface model and compared to a reference volume to detect edema.

The device 1 comprises a processor 5. The processor 5 may be, but is not necessarily limited to, a general-purpose processor (e.g. adapted for executing a program code), such as a central processing unit, a graphics processing unit, a cell processor, and the like. The processor 5 may equally refer to an application-specific integrated circuit or configurable hardware, such as a field-programmable gate array.

The processor 5 is adapted for determining, e.g. estimating, the total amount of fluid intake and total fluid outtake of the body, e.g. continuously, or at least periodically, taking a signal, e.g. a substantially real-time signal, provided by the body weight sensor 2 and a signal provided by the urine output sensor 4 into account.

The fluid intake may be determined based on a control signal provided to the fluid delivery device 3, and/or based on a feedback signal provided by the fluid delivery device. The total amount of fluid outtake may be determined based on changes in the signal provided by the body weight sensor, the signal provided by the urine output sensor and the determined fluid intake. While weighing the patient could, conceivably, provide sufficient data in combination with the known (e.g. directly controlled), determined or estimated fluid intake to determine the total fluid outtake, it is noted that, by taking also the urine output into account, a more accurate estimate of the fluid outtake can be achieved. Furthermore, in circumstances where fluid losses due to respiration and/or perspiration cannot be neglected, weighing the amount of produced urine alone is not sufficient to accurately estimate the total fluid losses of the body.

The processor 5 is adapted to estimate the amount of fluid lost (e.g. per unit of time; e.g. a continuous, real-time, periodic or frequent estimate) due to perspiration and respiration, taking the signal provided by the urine output sensor, the signal provided by the body weight sensor and a determined fluid intake into account. It is thereby clear that the fluid outtake due to perspiration and respiration will be equal to or smaller than the total fluid outtake of the body over a period of time. For example, this can be achieved by subtracting the urine output per unit of time and the fluid intake per unit of time from the change in body weight per unit of time. For example, other routes of fluid loss, e.g. except urination, perspiration and respiration, may be assumed to be negligible, e.g. fluid loss via defecation and/or hemorrhaging. A separate estimation of the fluid loss via perspiration and respiration may be particularly useful in procedures where the body is intentionally subjected to an elevated temperature, e.g. in a hyperthermia treatment. For example, this estimate of the respiratory/perspiratory fluid loss can be taken into account in a control algorithm to control thermal means, such as a heater and/or cooler, in a hyperthermia treatment.

It was found that under certain conditions, the signal provided by the body weight sensor, the signal provided by the urine output sensor and the determined fluid intake, optionally in combination with other sensor(s) to provide (a) further physiological parameter measurement(s), may enable a processor 5 to detect the formation of an edema in at least a part of the body. A non-limiting example includes a fluid build-up in the lungs which may be accompanied by a detectable increase in the partial pressure of $CO_2$ in the blood (pCOz) as well as blood acidification.

The processor 5 is adapted to control the fluid delivery device 3 so as to maintain a fluid balance in the body, in which the determined total fluid intake substantially equals the determined total fluid outtake. For example, the processor may implement a closed loop feedback system to minimize a difference between the fluid intake and the fluid outtake. For example, the processor may comprise a proportional-integral-derivative (PID) circuit. Accurate control of the fluid intake to aim for a balance with the fluid outtake may be particularly useful, e.g. even necessary, in a procedure where the body is intentionally subjected to an elevated temperature, e.g. a hyperthermia treatment. While the fluid balance may typically refer to a zero-fluid balance, i.e. a total intake equal to the total outtake, i.e. a zero sum of intake and outtake, this is not necessarily the case in all embodiments. For example, in an embodiment, the fluid balance may refer to an intentional predetermined difference between the total fluid intake and the total fluid outtake, e.g. to rehydrate or dehydrate the body at a safe rate and in a controlled manner.

The processor may be adapted for receiving a signal from the fluid retention detector 6 indicative of edema. For example, the signal provided by the fluid retention sensor may represent a length of a circumference of a body part, or may represent a strain of a strip that encloses, in use, a body part. Thus, the processor may compare the signal (or a change of the signal, e.g. a difference with respect to a reference measurement) to a threshold to detect abnormal fluid retention in the body part when the threshold is exceeded. Thus, a signal indicative of a body part circumference measurement may be analyzed to detect a substantial change in the circumference of the body part.

The processor may be adapted for analyzing images from a plurality of cameras to compute an image depth, and estimate a volume of the body based on the image depth. The processor may be adapted for receiving and analyzing laser scanning, e.g. LIDAR, data, and estimating a volume of the body based on the analyzed laser scanning data. It is an advantage of such approaches that fluid retention detection is not limited to one or a few locations on the body where, for example, a contour is monitored, but can be globally assessed by an estimate of (e.g. real-time) body volume.

The device may comprise an alarm 7, e.g. an audio and/or visual signal source, to alert an operator. The processor 5 may be adapted to activate the alarm 7 when the determined fluid intake is greater than or less than the determined fluid outtake by a predetermined margin, e.g. an absolute or relative margin.

The processor 5 may be adapted to activate the alarm 7 when a signal indicative of edema is received from the fluid retention detector 6.

The processor 5 may be adapted for controlling the fluid delivery device 3 in response to the signal indicative of edema, e.g. by blocking the delivery of fluid to the body when edema is detected. It will however be clear that approval by a member of the medical staff before blocking the delivery of fluid to the body may be preferred.

The device may comprise an output for outputting, e.g. displaying or in another way reporting, the determined total fluid outtake of the body and/or the specific fluid outtake due to perspiration and respiration.

In preferred embodiments according to the present invention, the body weight sensor 2 comprises at least three load cells, which are each integrated in or operably attached to said support structure for supporting the body in use of the device, wherein the support structure essentially forms a plane, being a flat surface in two dimensions X and Y. For ease of understanding, it is further assumed here below that the craniocaudal or longitudinal axis of the body is essentially located along the X-axis. The load cells are typically each integrated in or operably attached to a leg of the support structure contacting the ground. In embodiments, the body weight sensor comprises three, five or six load cells.

Preferably, the body weight sensor 2 comprises four load cells. Typically, said four legs are provided at the four corners of the support structure or near the four corners of the support structure.

In embodiments according to the present invention, the processor 5 is adapted for determining the center of gravity of the body held by the support structure by use of data provided by the at least three, preferably four, load cells. Such data may relate to the weights or forces applied to each leg and measured by each of the load cells. Typically, the processor is adapted to determine the (x,y)-coordinates of the center of gravity of the body in the plane formed by the support structure. The (x,y)-positions of the center of gravity can be determined by measuring the force or weight that the human body is causing at each of said four load cells. Preferably, the body of which the center of gravity is determined remains static during the period of monitoring the center of gravity. It can be assumed that a body which is brought under anesthesia or on which no external force is exerted meets this condition. However, this is not a necessary requirement, as a sudden movement of a patient, e.g. moving of an arm, would imply an abrupt shift of the center of gravity, which may be filtered out by e.g. a low-pass filter (LPF).

For the purpose of the invention, the term "center of gravity" refers herein to the unique point in an object or system in which all of the weight (or mass) of the object or system appears to be concentrated. For complex shapes like the human body, the center of gravity is typically concentrated near the umbilicus.

For illustration purposes and without limiting thereto, a method for determining the (x,y) positions of the center of gravity of a body for a body weight sensor having four load cells, may be based on the following equations:

$$x = (w_1{}^*x_1 + w_2{}^*x_2 + w_3{}^*x_3 + w_4{}^*x_4)/(w_1+w_2+w_3+w_4) \quad (1)$$

$$y = (w_1{}^*y_1 + w_2{}^*y_2 + w_3{}^*y_3 + w_4{}^*y_4)/(w_1+w_2+w_3+w_4) \quad (2)$$

wherein $W_i$ (i = 1 to 4) refers to the weight or force measured by the load cell having coordinates $(x_i, y_i)$ with reference to a reference point in the plane formed by the support structure serving as origin of X and Y axes.

In embodiments according to the present invention, the load cells have an accuracy of at least 50g, preferably at least 10g, more preferably at least 5g, and most preferably at least 1g. It has been found that such accuracy permits measuring a shift in center of gravity to a level which is considered accurate for the intended purposes.

In embodiments according to the present invention, the processor 5 is further provided with a low-pass filter (LPF), which can be used to filter noise or oscillations. For the purpose of the invention, the term "low-pass filter" refers to a filter that passes signals with a frequency lower than a selected cutoff frequency and attenuates signals with frequencies higher than the cutoff frequency. Preferably, a low-pass filter is used with a cutoff frequency of 0.1 Hz, or even 0.05 Hz. As such, the effect of breathing or heartbeats, which can otherwise have an oscillating effect on the center of gravity, can be filtered out.

In embodiments according to the present invention, the center of gravity is periodically weighed, e.g. continuously or at least frequently. For example, the body weight sensor 2 may be adapted for sampling the forces or weights at each of the load cells at a predetermined sampling frequency, e.g. at least once per minute, e.g. at least 6 times per minute, e.g. a sampling frequency in the range of 0.1 Hz to 1000 Hz, e.g. in the range of 1 Hz to 50 Hz.

Advantageously, determining the center of gravity enables observing changes to the fluid distribution in the body. More in particular, due to the short duration of measurements, it has been found that the main cause for any shifts in the (x,y)-position of the center of gravity of the body on the support structure, can be attributed to changes in local distribution of liquids in the body. Such changes may have several reasons.

A shift of the center of gravity towards the groin (therefore roughly in the X-direction) may be due to a buildup of urine in the bladder. As such, determining the center of gravity may indicate that a bladder catheter may be inserted incorrectly or may be obstructed, blocking urine output.

A shift of the center of gravity towards the thorax (therefore roughly in the X-direction), may indicate a buildup of liquid in the lungs. Hence, determining the center of gravity may detect the emergence of an edema in the lungs. It has furthermore been found that determining small shifts in the center of gravity suffice for detecting the emergence of edema.

A shift of the center of gravity towards the liver (roughly in both X and Y direction), may indicate internal hemorrhaging in the liver.

A shift of the center of gravity in the Y direction, may indicate the emergence of edema in one of the limbs, e.g. in an arm.

Hence, it will be appreciated by the skilled person that a sustained shift of the center of gravity, which shift may take minutes, may be used for detecting a possible dangerous condition for the human or animal body.

The alarm 7 may be provided to be activated to alert an operator should unusual shifts occur.

In embodiments according to the invention, the processor 5 is further adapted for comparing fluid delivered to the body over a first period of time to urine output measured for a second period of time, the second period of time have a time delay, typically 30-45 min, with respect to the first period of time. Indeed, it has been found that, regardless of fluid loss due to perspiration and respiration, the fluid balance is a dynamic process wherein the quantity of urine output is proportional to the fluid delivered to the body, considering a delay of typically 30-45 minutes. Hence, the processor 5 may be further adapted to monitor the urine output while taking into account the fluid delivered to the body. The processor may further be adapted to activate the alarm 7 in case an unexpected urine output is reported, taking into account said delivered fluid. A too low quantity of urine output measured vis-à-vis earlier delivered fluid may indicate the emergence of an edema in the body. Furthermore, it has been found that a too high quantity of urine measured may indicate dehydration of the body.

In a second aspect, the present invention relates to a system adapted for bringing a body into a prolonged state of increased temperature, comprising a monitoring device 1 in accordance with embodiments of the first aspect of the present invention. For example, the hyperthermia treatment system may be a system as described in EP 3372204, EP 3563814 and/or WO 2018/078188. A prolonged state of increased temperature may be at least 2 hours, at least 4 hours, at least 6 hours, at least 8 hours or at least 10 hours.

In a third aspect, the present invention relates to a method, e.g. a computer-implemented method, for monitoring the fluid balance of an animal or human body. The method comprises weighing, e.g. continuously or periodically, the body using a body weight sensor 2 and determining, e.g. continuously or periodically, the urine output of the body using a urine output sensor 4. The method comprises determining (e.g. by a processor 5) a total fluid outtake of the body, and optionally also a specific fluid outtake due to perspiration and respiration by taking the determined body weight and the determined urine output into account. The method comprises regulating (e.g. under control of the processor 5) a volume, flow velocity or flow rate of a fluid delivered to the body using a fluid delivery device 3 so as to maintain a predetermined fluid balance in the body, e.g. taking the determined total fluid outtake into account. Optionally, the method also comprises detecting a condition of edema in at least a part of the body based on a signal provided by a fluid retention detector 6. The method may comprise alerting an operator, using an alarm 7, when a fluid loss or gain exceeds a predetermined threshold and/or the condition of edema is detected. The method may also comprise blocking the delivery of fluid to the body when a fluid gain exceeds a predetermined threshold and/or the condition of edema is detected.

**Claims**

1. A monitoring device (1) for monitoring the fluid balance of an animal or human body, the device comprising:

   - a body weight sensor (2) for continuously or periodically weighing the body,
   - a fluid delivery device (3) for providing a controllable supply of fluid to the body,
   - a urine output sensor (4) for determining the urine output of the body, and
   - a processor (5),

   wherein said processor (5) is adapted for:

   - determining the total amount of fluid intake and total fluid outtake of the body, taking a signal provided by the body weight sensor (2) and a signal provided by the urine output sensor (4) into account;
   - determining a fluid outtake due to perspiration and respiration by taking a signal provided by the body weight sensor (2), a signal provided by the urine output sensor (4) and a determined fluid intake into account; and
   - providing a control signal to the fluid delivery device (3) to regulate a volume, flow velocity or flow rate of the fluid delivered to the body, so as to maintain a predetermined fluid balance in the body,

   wherein other routes of fluid loss, except urination, perspiration and respiration, are assumed to be negligible.

2. The device of claim 1, further comprising a fluid retention detector (6) for detecting edema in at least a part of the body, wherein the processor (5) is adapted for detecting a condition of edema based on a signal from the fluid retention detector (6).

3. The device according to claim 1 or claim 2, wherein the body weight sensor (2) comprises at least one load cell, integrated in or operably connected to a support structure for supporting the body in use of the device.

4. The device according to any of the previous claims, comprising an alarm (7) to alert an operator, wherein said processor (5) is adapted to activate the alarm when the condition of edema is detected.

5. The device according to any of the previous claims, wherein said processor (5) is adapted to control the fluid delivery device (3) so as to block the delivery of fluid to the body when the condition of edema is detected.

6. The device according to any of claims 2-5, wherein the fluid retention detector (6) comprises a range detector for determining a distance from at least a point of reference to a plurality of points on the surface of the body, said range detector forming a three-dimensional imaging system, wherein said processor (5) is adapted for determining a volume of the body based on data provided by the range detector.

7. The device according to claim 6, wherein said range detector comprises at least two cameras, and wherein said processor (5) is adapted for determining an image depth in images obtained by the cameras, for generating a three-dimensional contour model of the body based on the determined image depth and for determining a volume of the body based on the three-dimensional contour model as indicative of edema..

8. The device according to claim 6 or claim 7, wherein said range detector comprises at least one laser light source for illuminating the body, a sensor for measuring the reflected light, and a scanning system for scanning the laser light beam(s) emitted by the at least one laser light source over at least a part of the surface of the body while measuring the reflected light for a plurality of scanned points, wherein the processor (5) is adapted for collecting point cloud data of the body based on measured reflected light for the plurality of scanned points, for generating a three-dimensional contour model of the body based on the point cloud data and for determining a volume of the body based on the three-dimensional contour model as indicative of edema.

9. The device according to any of claims 2-8, wherein the fluid retention detector (6) comprises an element (61) for enclosing a part of the body and a sensor module (63), which is integrated in or operably attached to the element (61) and adapted for generating a signal indicative of a circumference of the body part.

10. The device according to claim 9, wherein said sensor module (63) comprises a capacitive, inductive or resistive strain sensor, and/or a linear variable differential transformer.

11. The device according to any of claims 2-10, wherein the fluid retention detector (6) comprises multiple parts for being positioned around different body parts and for generating separate signals indicative of swelling of each of the different body parts.

12. The device according to any of the previous claims, wherein the fluid delivery device (3) comprises an intravenous pump.

13. The device according to any of the previous claims, wherein said urine output sensor (4) comprises an optical sensing chamber, an ultrasonic transceiver, a measuring head, and/or a weighing system.

14. A hyperthermia treatment system comprising a monitoring device (1) in accordance with any of the previous claims.

**Patentansprüche**

1. Überwachungsvorrichtung (1) zum Überwachen des Flüssigkeitshaushalts eines tierischen oder menschlichen Körpers, wobei die Vorrichtung umfasst:

   - einen Körpergewichtssensor (2) zum kontinuierlichen oder periodischen Wiegen des Körpers,
   - eine Flüssigkeitszufuhrvorrichtung (3) zum Bereitstellen einer kontrollierbaren Flüssigkeitsversorgung des Körpers,
   - einen Urinabgabesensor (4) zum Bestimmen der Urinabgabe des Körpers, und
   - einen Prozessor (5),

   wobei der Prozessor (5) ausgelegt ist zum:

   - Bestimmen der Gesamtmenge der Flüssigkeitsaufnahme und der Gesamtflüssigkeitsabgabe des Körpers unter Berücksichtigung eines durch den Körpergewichtssensor (2) bereitgestellten Signals und eines durch den Urinausgabesensor (4) bereitgestellten Signals;
   - Bestimmen einer Flüssigkeitsabgabe aufgrund von Schwitzen und Atmung unter Berücksichtigung eines durch den Körpergewichtssensor (2) bereitgestellten Signals, eines durch den Urinausgabesensor (4) bereitgestellten Signals und einer bestimmten Flüssigkeitsaufnahme; und
   - Übermitteln eines Steuersignals an die Flüssigkeitszufuhrvorrichtung (3), um ein Volumen, eine Strömungsgeschwindigkeit oder eine Strömungsrate der dem Körper zugeführten Flüssigkeit zu regulieren, um einen zuvor festgelegten Flüssigkeitshaushalt im Körper aufrecht zu erhalten,

   wobei andere Wege des Flüssigkeitsverlustes, mit Ausnahme von Urinieren, Schwitzen und Atmen, als vernachlässigbar angenommen werden.

2. Vorrichtung nach Anspruch 1, die des Weiteren einen Flüssigkeitsretentionsdetektor (6) zum Detektieren eines Ödems in mindestens einem Teil des Körpers umfasst, wobei der Prozessor (5) zum Detektieren eines Ödemzustands auf der Grundlage eines Signals von dem Flüssigkeitsretentionsdetektor (6) ausgelegt ist.

3. Vorrichtung nach Anspruch 1 oder Anspruch 2, wobei der Körpergewichtssensor (2) mindestens eine Wägezelle umfasst, die in eine Stützstruktur integriert oder mit einer Stützstruktur wirkverbunden ist, die den Körper bei der Verwendung der Vorrichtung stützt.

4. Vorrichtung nach einem der vorangehenden Ansprüche, die einen Alarm (7) umfasst, um einen Bediener zu alarmieren, wobei der Prozessor (5) dafür ausgelegt ist, den Alarm zu aktivieren, wenn der Ödemzustand detektiert wird.

5. Vorrichtung nach einem der vorangehenden Ansprüche, wobei der Prozessor (5) dafür ausgelegt ist, die Flüssigkeitszufuhrvorrichtung (3) so zu steuern, dass die Versorgung des Körpers mit Flüssigkeit blockiert wird, wenn der Ödemzustand detektiert wird.

6. Vorrichtung nach einem der Ansprüche 2-5, wobei der Flüssigkeitsretentionsdetektor (6) einen Entfernungsdetektor zum Bestimmen einer Distanz von mindestens einem Bezugspunkt zu mehreren Punkten auf der Oberfläche des Körpers umfasst, wobei der Entfernungsdetektor ein dreidimensionales Abbildungssystem bildet, wobei der Prozessor (5) zum Bestimmen eines Volumens des Körpers auf der Grundlage von durch den Entfernungsdetektor bereitgestellten Daten ausgelegt ist.

7. Vorrichtung nach Anspruch 6, wobei der Entfernungsdetektor mindestens zwei Kameras umfasst, und wobei der Prozessor (5) dafür ausgelegt ist, eine Bildtiefe in durch die Kameras erhaltenen Bildern zu bestimmen, ein dreidimensionales Konturmodell des Körpers auf der Grundlage der bestimmten Bildtiefe zu erzeugen und ein Volumen des Körpers auf der Grundlage des dreidimensionalen Konturmodells als Anzeichen für ein Ödem zu bestimmen.

8. Vorrichtung nach Anspruch 6 oder 7, wobei der Entfernungsdetektor umfasst: mindestens eine Laserlichtquelle zum Beleuchten des Körpers, einen Sensor zum Messen des reflektierten Lichts, und ein Abtastsystem zum Abtasten des einen oder der mehreren durch die mindestens eine Laserlichtquelle ausgesendeten Laserlichtstrahlen über mindestens einen Teil der Oberfläche des Körpers, während das reflektierte Licht für mehrere abgetastete Punkte gemessen wird, wobei der Prozessor (5) zum Sammeln von Punktwolkendaten des Körpers auf der Grundlage von gemessenem reflektiertem Licht für die mehreren abgetasteten Punkte, zum Generieren eines dreidimensionalen Konturmodells des Körpers auf der Grundlage der Punktwolkendaten, und zum Bestimmen eines Volumens des Körpers auf der Grundlage des dreidimensionalen Konturmodells als Anzeichen für ein Ödem ausgelegt ist.

9. Vorrichtung nach einem der Ansprüche 2-8, wobei der Flüssigkeitsretentionsdetektor (6) umfasst: ein Element (61) zum Umschließen eines Teils des Körpers, und ein Sensormodul (63), das in das Element (61) integriert oder an dem Element (61) angebracht ist und dafür ausgelegt ist, ein Signal zu generieren, das einen Umfang des Körperteils angibt.

10. Vorrichtung nach Anspruch 9, wobei das Sensormodul (63) einen kapazitiven, induktiven oder resistiven Dehnungssensor und/oder einen linearen variablen Differenzialtransformator umfasst.

11. Vorrichtung nach einem der Ansprüche 2-10, wobei der Flüssigkeitsretentionsdetektor (6) mehrere Teile umfasst, die dafür gedacht sind, um verschiedene Körperteile herum positioniert zu werden und separate Signale zu generieren, die eine Schwellung jedes der verschiedenen Körperteile angeben.

12. Vorrichtung nach einem der vorangehenden Ansprüche, wobei die Flüssigkeitszufuhrvorrichtung (3) eine intravenöse Pumpe umfasst.

13. Vorrichtung nach einem der vorangehenden Ansprüche, wobei der Urinabgabesensor (4) eine optische Messkammer, einen Ultraschall-Transceiver, einen Messkopf und/oder ein Wiegesystem umfasst.

14. Hyperthermie-Behandlungssystem, das eine Überwachungsvorrichtung (1) nach einem der vorangehenden Ansprüche umfasst.

**Revendications**

1. Dispositif de surveillance (1) pour surveiller l'équilibre fluidique d'un corps animal ou humain, le dispositif comprenant :

   - un capteur de poids corporel (2) pour peser continuellement ou périodiquement le corps,
   - un dispositif de délivrance de fluide (3) pour fournir au corps une alimentation en fluide pouvant être commandée,
   - un capteur de production d'urine (4) pour déterminer la production d'urine du corps, et
   - un processeur (5),

   dans lequel ledit processeur (5) est conçu pour :

   - déterminer la quantité totale d'apport de fluide et de sortie totale de fluide du corps, en prenant en compte un signal fourni par le capteur de poids corporel (2) et un signal fourni par le capteur de production d'urine (4) ;
   - déterminer une sortie de fluide due à la transpiration et à la respiration en prenant en compte un signal fourni par le capteur de poids corporel (2), un signal fourni par le capteur de production d'urine (4) et un apport de fluide déterminé ; et
   - fournir un signal de commande au dispositif de délivrance de fluide (3) pour réguler un volume, une vitesse d'écoulement ou un débit du fluide délivré au corps, de manière à maintenir un équilibre fluidique prédéterminé dans le corps,

   dans lequel d'autres voies de perte de fluide, à l'exception de la miction, de la transpiration et de la respiration, sont supposées être négligeables.

2. Dispositif selon la revendication 1, comprenant en outre un détecteur de rétention de fluide (6) pour détecter un œdème dans au moins une partie du corps, dans lequel le processeur (5) est conçu pour détecter une condition d'œdème sur la base d'un signal provenant du détecteur de rétention de fluide (6).

3. Dispositif selon la revendication 1 ou la revendication 2, dans lequel le capteur de poids corporel (2) comprend au moins une cellule de charge, intégrée dans ou connectée de manière opérationnelle à une structure de support pour supporter le corps lors de l'utilisation du dispositif.

4. Dispositif selon l'une quelconque des revendications précédentes, comprenant une alarme (7) pour alerter un opérateur, dans lequel ledit processeur (5) est conçu pour activer l'alarme lorsqu'une condition d'oedème est détectée.

5. Dispositif selon l'une quelconque des revendications précédentes, dans lequel ledit processeur (5) est conçu pour commander le dispositif de distribution de fluide (3) de manière à bloquer la délivrance de fluide au corps lorsqu'une condition d'oedème est détectée.

6. Dispositif selon l'une quelconque des revendications 2 à 5, dans lequel le détecteur de rétention de fluide (6) comprend un détecteur de portée pour déterminer une distance depuis au moins un point de référence jusqu'à une pluralité de points sur la surface du corps, ledit détecteur de portée formant un système d'imagerie tridimensionnelle, dans lequel ledit processeur (5) est conçu pour déterminer un volume du corps sur la base de données fournies par le détecteur de portée.

**7.** Dispositif selon la revendication 6, dans lequel ledit détecteur de portée comprend au moins deux appareils de prise de vues, et dans lequel ledit processeur (5) est conçu pour déterminer une profondeur d'image dans des images obtenues par les appareils de prise de vues, pour générer un modèle de contour tridimensionnel du corps sur la base de la profondeur d'image déterminée et pour déterminer un volume du corps sur la base du modèle de contour tridimensionnel comme étant indicatifs d'œdème.

**8.** Dispositif selon la revendication 6 ou la revendication 7, dans lequel ledit détecteur de portée comprend au moins une source de lumière laser pour éclairer le corps, un capteur pour mesurer la lumière réfléchie, et un système de balayage pour balayer le ou les faisceaux de lumière laser émis par l'au moins une source de lumière laser sur au moins une partie de la surface du corps tout en mesurant la lumière réfléchie pour une pluralité de points balayés, dans lequel ledit processeur (5) est conçu pour collecter des données de nuage de points du corps sur la base de la lumière réfléchie mesurée pour la pluralité de points balayés, pour générer un modèle de contour tridimensionnel du corps sur la base des données de nuage de points et pour déterminer un volume du corps sur la base du modèle de contour tridimensionnel comme étant indicatifs d'œdème.

**9.** Dispositif selon l'une quelconque des revendications 2 à 8, dans lequel le détecteur de rétention de fluide (6) comprend un élément (61) pour enfermer une partie du corps et un module capteur (63), qui est intégré ou fixé de manière opérationnelle à l'élément (61) et conçu pour générer un signal indicatif d'une circonférence de la partie de corps.

**10.** Dispositif selon la revendication 9, dans lequel ledit module capteur (63) comprenant un capteur de contrainte capacitif, inductif ou résistif, et/ou un transformateur différentiel variable linéaire.

**11.** Dispositif selon l'une quelconque des revendications 2 à 10, dans lequel le détecteur de rétention de fluide (6) comprenant plusieurs parties à positionner autour de différentes parties du corps et pour générer des signaux distincts indicatifs de gonflement de chacune des différentes parties de corps.

**12.** Dispositif selon l'une quelconque des revendications précédentes, dans lequel le dispositif de délivrance de fluide (3) comprend une pompe intraveineuse.

**13.** Dispositif selon l'une quelconque des revendications précédentes, dans lequel ledit capteur de production d'urine (4) comprend une chambre de détection optique, un émetteur-récepteur d'ultrasons, une tête de mesure, et/ou un système de pesée.

**14.** Système de traitement par hyperthermie comprenant un dispositif de surveillance (1) selon l'une quelconque des revendications précédentes.

1

**FIG 1**

**FIG 2**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4291692 A
- US 4449538 A
- US 5522805 A
- US 5876353 A
- US 2018085510 A1
- US 2001051788 A
- US 4504263 A

- US 4448207 A
- US 4658834 A
- US 5176148 A
- EP 3185757 A
- EP 3372204 A
- EP 3563814 A
- WO 2018078188 A